(19) Europäisches Patentamt
European Patent Office
Office européen des brevets

(11) Publication number: **0 061 768**
**B1**

(12) **EUROPEAN PATENT SPECIFICATION**

(45) Date of publication of patent specification: **14.05.86**

(51) Int. Cl.⁴: **C 07 K 5/06,** A 61 K 37/02

(21) Application number: **82102653.1**

(22) Date of filing: **29.03.82**

(54) **N-amido substituted dipeptides.**

(30) Priority: **30.03.81 US 249053**

(43) Date of publication of application: **06.10.82 Bulletin 82/40**

(45) Publication of the grant of the patent: **14.05.86 Bulletin 86/20**

(84) Designated Contracting States: **AT BE CH DE FR GB IT LI LU NL SE**

(56) References cited:
**EP-A-0 012 401**
**GB-A-2 054 586**

(73) Proprietor: **USV PHARMACEUTICAL CORPORATION**
**1 Scarsdale Road**
**Tuckahoe New York (US)**

(72) Inventor: **Skiles, Jerry W.**
**1 Consulate Drive**
**Tuckahoe New York (US)**
Inventor: **Youssefyeh, Raymond D.**
**435 Martling Av.**
**Tarrytown New York (US)**
Inventor: **Suh, John T.**
**59 Stanwich Road**
**Greenwich Connecticut (US)**
Inventor: **Jones, Howard**
**75 Briarcliff Woods**
**Ossining New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new chemical compounds having valuable pharmaceutical activity. It particularly relates to N-substituted-amido-amino acids possessing hypertensive and angiotensin converting enzyme inhibitory (ACEI) activity and having the structure

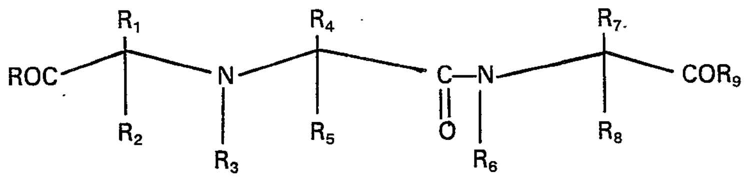

I

wherein

R and $R_9$ are independently hydroxy, lower alkoxy, lower alkenoxy, di(lower alkyl) amino-lower alkoxy, hydroxy-lower alkoxy, acylamino-lower alkoxy, acyloxy-lower alkoxy, aryloxy, aryloxy-lower alkoxy, amino, lower alkylamino, dilower alkylamino, hydroxyamino, or aryl-lower alkylamino;

$R_1$, $R_2$, $R_3$, $R_4$, $R_5$, $R_7$, and $R_8$ are independently hydrogen, alkyl, alkenyl or alkynyl containing up to 20 carbon atoms, aryl or aryl-lower alkyl containing up to 12 carbon atoms, heterocyclic or heterocyclic-lower alkyl having from 6 to 12 carbon atoms or cycloalkyl or cycloalkyl alkyl containing up to 20 carbon atoms;

$R_6$ is heterocyclic and heterocyclic lower alkyl;

$R_2$ and $R_3$ taken together with the carbon and nitrogen to which they are respectively attached and $R_3$ and $R_5$ taken together with the nitrogen and carbon to which they are respectively attached form an N-heterocycle containing from 3 to 5 carbon atoms or 2 to 4 carbon atoms and a sulfur atom; and

salts thereof, especially pharmaceutically acceptable salts with acids or bases.

From EP—A—12 401 carboxyalkyl dipeptide derivatives which are useful as antihypertensives are known having a similar chemical structure:

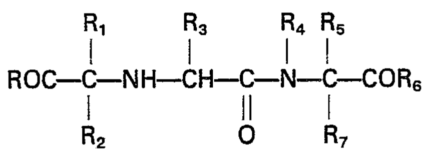

wherein, however, $R_4$ (corresponding to $R_6$ in the above formula I) is hydrogen or alkyl instead of a heterocyclic group.

From GB—A—2 054 586 antihypertensive amides having the following structure are known:

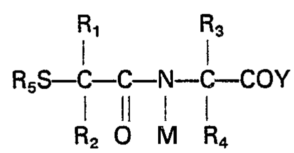

wherein M (corresponding to $R_6$ in the above formula I) can be a heterocyclic group, which however contains —S— and thus being a mercaptan compound.

However, in both cases the ACEI-activity is low as has been shown by suitable experiments. Even an expert in this technical field could not predict how the structure of compounds possessing the desired high ACEI-activity should be.

Surprisingly it has now been found that N-substituted amido-amino acids of the above formula I possess not only a high hypertensive activity, but also a particular high angiotensin converting enzyme inhibitory (ACEI) activity.

The meanings of the substituents R—$R_9$ in the above formula I are in detail as follows.

The alkyl groups per se or when present as substituents are preferably lower alkyl containing from 1 to 6 carbon atoms and may be straight chain or branched. These groups include methyl ethyl, propyl, iso-propyl, butyl, isobutyl, amyl, hexyl and the like.

The alkenyl and alkynyl groups per se or when present as substituents preferably contain from 2 to 6 carbon atoms and may be straight chain or branched. These groups include vinyl, propenyl, alkyl, isopropenyl, ethynyl and the like.

The alkyl, alkenyl, and alkynyl groups may carry substituents such as hydroxy, lower alkoxy, thio, lower alkylmercapto, amino, lower alkylamino, di(lower alkyl) amino, halogen, and nitro.

The aralkyl and heterocyclic-alkyl groups include benzyl, phenethyl, naphthylmethyl, indolylethyl, indanylmethyl, indanylethyl and the like.

The heterocyclic groups include saturated, partially saturated, and aromatic ring systems containing one or more atoms other than carbon, as well as heterocyclic lower alkyl. These heterocyclic groups

include pyrrole, pyrroline, pyrrolidine, pyridine, dihydropyridine, piperidine, morpholine, thiomorpholine, imidazole, imidazoline, imidazolidine, furan, furfuryl, thiophene, benzimidazole, thiazole, thiazoline, thiazolidine, indole, quinoline, isoquinoline, tetrahydroquinoline, tetrahydroisoquinoline, and the like.

The heterocyclic groups may carry one or more substituents such as lower alkyl, lower alkenyl, lower alkynyl, hydroxy, lower alkoxy, amino, lower alkylamino, di(lower alkyl)amino, thiol, lower alkylmercapto, hydroxy-lower alkyl, amino-lower alkyl, thio-lower alkyl, nitro, halogen, trifluoromethyl, methylene-dioxy, ureido, or guanidino.

The acyl groups are preferably lower alkanoyl containing from 1 to 6 carbon atoms and benzoyl.

The halogen group may be fluorine, chlorine, bromine and iodine.

Suitable acid addition salts may be formed from inorganic acids such as hydrochloric, sulfuric and phosphoric, and organic acids such as acetic, lactic, citric, malic, maleic, fumaric, succinic, benzoic, hydroxybenzoic aminobenzoic, nicotinic, benzenesulfonic and the like.

Suitable basic salts may include the salts of alkali and alkali earth metals such as sodium, lithium, potassium, magnesium and calcium, as well as iron and salts of ammonia and amines, and quaternary ammonium salts.

The compounds of the present invention may contain one (1) or more asymmetric carbon atoms and may exist in racemic and optically active forms. All of these forms are contemplated to be within the scope of the present invention.

The compounds of the present invention are prepared by amide-forming reaction of a compound of the formula

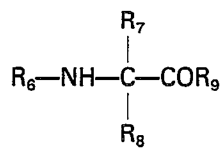

II

with an acylating derivative of an acid of the formula:

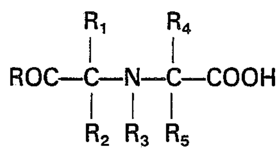

III

to give the desired compound.

As an alternative approach, a dipeptide of the structure

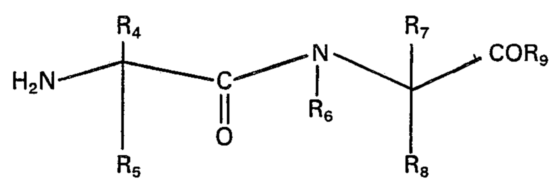

IV

is reacted with an α keto-acid or ester of the structure

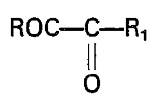

V

to form the corresponding imine and the imine is reduced to give a compound of formula I in which $R_2$ and $R_3$ are each H.

As a further reaction, the present new compounds can be prepared by reaction of a compound of the formula:

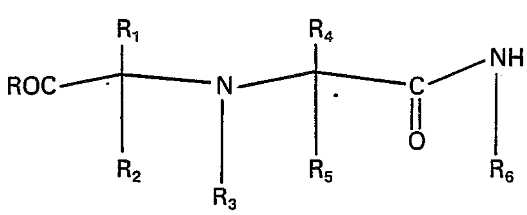

VI

with an α-halo compound of the formula

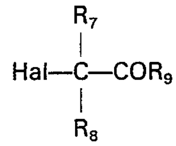

VII

by cleavage of hydrogen halide.

The aforementioned condensation reaction to form imines with subsequent hydrogenation can be conveniently carried out in a single reaction zone by the expediency of mixing the α-keto acid or derivative with the reactive compound of formula IV under hydrogenation conditions. For practical purposes, the aforesaid reactants can be hydrogenated over noble metal catalyst such as palladium, platinum, rhodium, ruthenium, and the like and the two stages occur under such conditions to produce the desired end-product.

As in any organic reaction solvents can be employed such as methanol, ethanol, propanol, acetone, tetrahydrofuran, dioxane, dimethylformamide, diethylacetamide, and the like. The reaction is normally effected at or near room temperature, although temperatures from 0°C up to the reflux temperature of the reaction mixture can be employed.

In the above sequence of reactions, R—$R_9$ are the same as described above and Hal is halogen. Preferably, R and $R_9$ are hydrogen or lower alkyl, $R_2$, $R_5$, $R_7$ and $R_8$ are hydrogen, $R_1$ and $R_4$ are lower alkyl, $R_3$ is hydrogen, and $R_6$ is indole or thienyl.

The amide forming conditions referred to herein involve the use of known derivatives of the described acids, such as the acyl halides, anhydrides, mixed anhydrides, lower alkyl esters, carbodiimides, carbonyl diimidazoles, and the like. The reactions are carried out in organic solvents such as acetonitrile, tetrahydrofuran, dioxane, acetic acid, methylene chloride, ethylene chloride and similar such solvents. The amide forming reaction will occur at room temperature or at elevated temperature. The use of elevated temperature is for convenience in that it permits somewhat shortened reaction periods. Temperatures ranging from 0°C up to the reflux temperature of the reaction system can be used. As a further convenience the amide forming reaction can be effected in the presence of a base such as tertiary organic amines, e.g., trimethylamine, pyridine, picolines and the like, particularly where hydrogen halide is formed by the amide-forming reaction, e.g., acyl halide and amino compound. Of course, in those reactions where hydrogen halide is produced, any of commonly used hydrogen halide acceptors can also be used.

In the condensation of an alpha haloacid derivative of formula VII herein, similar reaction conditions, solvents and hydrogen halide acceptors can be used as for amide formation.

Various substituents on the present new compounds e.g., as defined for $R_8$, can be present in the starting compounds or added after formation of the amide products by the known methods of substitution or conversion reactions. Thus, the nitro group can be added to the final product by nitration of the aromatic ring and the nitro group converted to other groups, such as amino by reduction, and halo by diazotization of the amino groups and replacement of the diazo group. Other reactions can be effected on the formed amide product. Amino groups can be alkylated to form mono and dialkylamino groups, mercapto and hydroxy groups can be alkylated to form corresponding ethers. Thus, substitution or alteration reactions can be employed to provide a variety of substituents throughout the molecule of the final products. Of course, reactive groups where present should be protected by suitable blocking groups during any of the aforesaid reactions particularly the condensation reactions to form the amide linkages.

The acid and base salts of the present new compounds can be formed using standard procedures. Often, they are formed *in situ* during the preparation of the present new amido amino acids.

The present compounds obviously exist in stereo-isomeric forms and the products obtained thus can be mixtures of the isomers, which can be resolved. Alternatively, by selection of specific isomers as starting compounds, the preferred stereoisomer can be produced. Therefore, the preferred forms, where each asymmetric center (chairal center) is S-configuration, are preferably prepared by the stereospecific route rather than attempting resolution of mixtures of isomers. The compounds in which the S-configuration exists at all asymmetric centers are the most active; those in which the R-configuration exists are of less activity; and those where both R- and S-configurations exist are of intermediate activity.

The invention is further illustrated by the following examples.

Example 1

A. t-Butyl N-[2-(3-indolyethyl)]-glycinate

Tryptamine hydrochloride (100 g, 624 mmols) was added to a mixture of acetonitrile (1 l) and concentrated ammonium hydroxide (48.8 ml). *Tert*-butylbromoacetate (101 g, 518 mmols) in acetonitrile was added dropwise over one hour. The reaction was allowed to stir overnight at room temperature. The solvent was evaporated and the residue was portioned between ethyl acetate and aqueous ammonium hydroxide. The layers were separated and the organic layer was washed with water and brine, dried over magnesium sulfate, filtered and evaporated. The crude product was crystallized from n-hexane/ether to give tan crystals (118 g, 83%), m.p. 88—90°C.

B. t-Butyl N-(1-(S)-carbethoxyethyl)-L-alanyl-N-[2-(3-indolyethyl)]-glycinate

N-(1-(S)-Carbethoxyethyl)-L-alanine (3.7 g, 0.02 mol) and 1,1′-carbonyldiimidazole (3.6 g, 0.022 mol) were added to dry tetrahydrofuran (30 ml). The resulting mixture was refluxed under nitrogen for thirty minutes. To the resulting solution was added t-butyl N - [2 - (3 - indolyethyl)] - glycinate (5.5 g, 0.02 mol) in tetrahydrofuran (15 ml). The resulting mixture was refluxed for two and a half hours. The solvent was evaporated and the residue was dissolved in chloroform. The chloroform was washed twice with water, dried over magnesium sulfate, filtered, and evaporated. The crude product was chromatographed on silica-gel ($CHCL_3$) to give the pure product (8 g, 83%) as a light auburn oil. The product was characterized as its hydrochloride salt; m.p. 140°C.

Example 2

A. Benzyl 2-bromopropionate

2-Bromopropionic acid (750 g, 4.90 moles) and benzyl alcohol (600 g, 3.55 moles) were dissolved in methylene chloride (1500 ml). To the resulting solution was added concentrated sulfuric acid (10 ml). The resulting solution was heated to a gentle reflux for two days, water separating during this time. Water (500 ml) was added and the layers were separated. The methylene chloride was washed with saturated sodium bicarbonate and the layers were separated. The organic layer was washed with water, dried over magnesium sulfate, filtered and evaporated to give a colorless oil which was vacuum distilled to give the pure product as a colorless oil (742 g, 86%).

B. Benzyl N-(1-(S)-ethoxycarbonyl-3-methylbutyl)-L-alanyl-N-[2-(3-indolylethyl)]-glycinate

N - (1 - (S) - Ethoxycarbonyl - 3 - methylbutyl) - L - alanine (2.31 g, 0.01 mol) and 1,1′-carbonyldiimidazole (2.3 g, 0.0142 mol) were added to dry tetrahydrofuran (40 ml). The resulting mixture was refluxed for fifteen minutes. To the resulting solution was added portionwise benzyl N-(2-indolylethyl)-glycinate (3.08 g, 0.01 mole) dissolved in THF (15 ml). The resulting solution was refluxed for two-and-a-half hours. The solvent was evaporated and the residue was dissolved in ether. The ether was washed three times with water. The ether was dried over magnesium sulfate, filtered, and evaporated to give the crude product. The crude product was chromatographed on silica-gel (ether) to give the pure product (3.8 g, 72%) as a light-colored oil which was a mixture of diastereomers.

Example 3

A. t-Butyl N-carbobenzyloxy-(L-valyl)-N-[2-(3-indolylethyl)]-glycinate

t-Butyl N-(2-indolylethyl)-glycinate (12.0 g, 43.8 mmols) was dissolved in methylene chloride (400 ml) and the solution was cooled in an ice bath. Dicyclohexylcarbondiimide (8.2 g, 39.8 mmols) in a small amount of methylene chloride was added. N-Carbobenzyloxy-L-valine (10 g, 39.8 mmols) in a small amount of methylene chloride was added dropwise, N-carbobenzyloxy-L-valine (10 g, 39.8 mmols) in a small amount of methylene chloride was added dropwise. The reaction was stirred with external cooling for 15 minutes and then at room temperature overnight. Precipitated dicyclohexylurea was filtered and washed with a small amount of methylene chloride. The filtrate was evaporated and ether was added to the residue to precipitate more dicyclohexylurea. The dicyclohexylurea was filtered and the filtrate was evaporated to afford the crude product as a tan gum. The crude product was purified by HPLC using ethyl acetate/methylene chloride (10:90) as eluent to give the pure product as a light tan powder (23.8 g, 91.2%).

B. N-Carbobenzyloxy-(L-valyl)-N-[2-(3-indolylethyl)]-glycine

t-Butyl N - carbobenzyloxy(L - valyl) - N - [2 - (3 - indolylethyl)] - glycinate (22.5 g, 44.3 mmols) was dissolved in anisole (47.9 g, 443.2 mmols) and then trifluoroacetic acid (101.1 g, 886.5 mmols) was added. The resulting solution was stirred for three hours at room temperature. Trifluoroacetic acid was removed at 30°C under vacuum. The dark brown oil was dissolved in ether and the organic extract was washed twice with water and dried over magnesium sulfate. The ether was filtered and evaporated to afford the crude product as a dark oil. The crude product was purified by HPLC eluting with acetic acid/ethyl acetate/*n*-hexane (2:39:59) to afford the pure product as a pale yellow powder (11.1 g).

Example 4

Benzyl N-(1-(S)-ethoxycarbonyl-2-phenylethyl)-L-alanyl-N-[2(3-indolylethyl)]-glycinate

[N-(Ethyl 2 - phenyl - 1 - (s) - propionate] - L - alanine (2.65 g, 10.0 mmols) and 1,1′-carbonyldiimidazole (2.0 g, 0.0123 mols) were added to dry tetrahydrofuran (40 ml). The resulting mixture was refluxed for fifteen minutes under nitrogen. To the resulting solution was added portionwise benzyl N - [2(3 - indolylethyl)] - glycinate (3.5 g, 11.4 mmols) in a small amount of THF (20 ml). The resulting solution was refluxed for three hours. The solvent was evaporated and the residue was dissolved in ether. The ether was washed twice with water, dried over magnesium sulfate, filtered and evaporated to afford an auburn oil. The crude product was chromatographed on silica-gel to afford the pure product as a yellow oil (4.5 g, 81%) which was a mixture of diastereomers.

Example 5

A. Ethyl N-carbobenzyloxy (L-phenylalanyl-N-[2(3-indolylethyl)]-glycinate

Ethyl N - [2 - (3 - indolylethyl)] - glycinate (17.3 g, 0.0703 mol) was dissolved in methylene chloride (400 ml) and the resulting solution was cooled in an ice bath. Dicyclohexylcarbodiimide (16.0 g, 0.078 mol) in a small amount of methylene chloride (25 ml) was added portionwise. N-carbobenzyloxy-L-phenylalanine (21.04 g, 0.0703 mol) was added portionwise. The reaction was stirred with external cooling for fifteen minutes and then for two-and-a-half hours at room temperature. Precipitated dicyclohexylurea was filtered and washed with ether. The filtrate was evaporated. The residue was dissolved in ether and washed with 10% HCL, saturated $NaHCO_3$, water and dried over magnesium sulfate. Filtration and evaporation of the solvent gave a tan viscous oil (33.7 g, 95%).

B. L-Phenylalanyl-N-[2(3-indolylethyl)]-glycine

N - Carbobenzyloxy - L - phenylalanyl - N - [2(3 - indolylethyl)] - glycine (1.3 g, 0.0026 mol) was added to a saturated solution of anhydrous HBr in glacial acetic acid (20 ml) which had been chilled in an ice bath. The resulting solution was stirred for fifteen minutes with external cooling and then for forty-five minutes at room temperature.

Most of the acetic acid was evaporated and ether was added to the residue to precipitate the hydrobromide of the product. The hydrobromide was filtered and washed with ether to afford tan crystals (0.85 g, 70.8%); m.p. 140°.

Example 6

A. Ethyl N-carbobenzyloxy-L-alanyl-N-(3-thienyl)glycinate

A methylene chloride solution of N-carbobenzyloxy-L-alanine and ethyl (3-thienyl) glycinate was treated with N,N-dicyclohexylcarbodiimide as in Example 5A. The product was applied to silica-gel to afford ethyl N - carbobenzyloxy - L - alanyl - N - (3 - thienyl)glycinate.

B. N-Carbobenzyloxy-L-alanyl-N-(3-thienyl)glycinine

An ethanolic solution of ethyl N - carbobenzyloxy - L - alanyl - N - (3 - thienyl) glycinate was treated with two equivalents of potassium hydroxide to yield N - carbobenzyloxy - L - alanyl - N - (3 - thienyl) glycine.

C. L-Alanyl-N-(3-thienyl)glycine

In a manner described in Example 5B, N - carbobenzyloxy - L - alanyl - N - (3 - thienyl) glycine was treated with anhydrous hydrogen bromide in acetic acid to yield the hydrobromide salt of L - alanyl - N - (3 - thienyl) glycine.

Example 7

N-(1-Carboxy-3-phenylpropyl)-L-alanyl-N-(3-thienyl)glycine

2-Oxo-4-phenylbutyric acid and L - alanyl - N - (3 - thienyl) - glycine are condensed in the presence of sodium cyanoborohydride to yield N - (1 - carboxy - 3 - phenylpropyl) - L - alanyl - N - (3 - thienyl)glycine.

Example 8

A. N-Carbobenzyloxy-L-isoleucyl-N-(3-pyridyl)glycinine

An ethanolic solution of ethyl N-carbobenzyloxy - L - isoleucyl - N - (3 - pyridyl)glycinate was treated with potassium hydroxide to yield N - carbobenzyloxy - L - isoleucyl - N - (3 - pyridyl)glycine.

B. Ethyl N-Carbobenzyloxy-L-isoleucyl-N-(3-pyridyl)glycinate

A methylene chloride solution of N-carbobenzyloxy-L-isoleucine and ethyl N-(3-pyridyl)-glycinate was treated with N,N-dicyclohexylcarbodiimide as in Example 5A. Purification of the product was accomplished by chromatography on silica-gel.

C. L-Isoleucyl-N-(3-pyridyl)glycinate

In a manner described in Example 5B, N - carbobenzyloxy - L - isoleucyl - N - (3 - pyridyl) - glycine was treated with anhydrous hydrogen bromide in acetic acid to yield L - isoleucyl - N - (3 - pyridyl) - glycine hydrobromide.

Example 9

N-[1-(S)-Ethoxycarbonyl-3-methylbutyl]-L-isoleucyl-N-(3-pyridyl)glycine

Ethyl 4-methyl-2-oxopentanoate and L - isoleucyl - N - (3 - pyridyl) - glycine were condensed in the presence of sodium cyanoborohydride to yield N - [1 - (S) - ethoxycarbonyl - 3 - methyl - butyl] - L - isoleucyl - N - (3 - pyridyl)glycine.

Example 10

A. N-Carbobenzyloxy-L-leucyl-N-(2-ethylmorpholine)glycine

An ethanolic solution of ethyl N - carbobenzyloxy - L - leucyl - N - (2 - ethylmorpholine)glycinate

was treated with potassium hydroxide to yield N - carbobenzyloxy - L - leucyl - N - (2 - ethylmorpholine)glycine.

B. Ethyl N-carbobenzyloxy-L-leucyl-N-(2-ethylmorpholine)glycine

A methylene chloride solution of N-carbobenzyloxy-L-leucine and ethyl N-(2-ethylmorpholine)glycinate was treated with N,N-dicyclohexylcarbodiimide as in Example 5A. The product was purified by chromatography (silica-gel) to yield ethyl N - carbobenzyloxy - L - leucyl - N - (2 - ethylmorpholine)glycinate.

Example 11

Benzyl N-(1-(S)-ethoxycarbonyl-2-phenylethyl) alanyl-N-(2-benzothiazole)glycinate

Benzyl N - [1 - (S) - ethoxycarbonyl - 2 - phenylethyl]alanine and 1,1-carbonyldiimidazole were added to dry tetrahydrofuran. The resulting mixture was refluxed for fifteen minutes. To the resulting solution was added portionwise benzyl N - (2 - benzothiazole) - glycinate. The resulting mixture was refluxed for three hours. The product was purified in a manner as described in Example 1B.

Example 12

A. Benzyl N-[1-(S)-ethoxycarbonylethyl]alanyl-N-(2-pyrimidyl)glycinate

N - [1 - (S) - Ethoxycarbonylethyl] alanine and 1,1′-carbonyldiimidazole were added to dry tetrahydrofuran. The resulting mixture was refluxed for fifteen minutes. To the resulting solution was added benzyl N-(2-pyrimidyl)glycinate. The resulting mixture was refluxed for three hours. The product was purified by HPLC chromatography to yield benzyl N - [1 - (S) - ethoxycarbonylethyl] - alanyl - N - (2 - pyrimidyl)glycinate.

B. N-[1-(S)-Ethoxycarbonylethyl]alanyl-N-(2-pyrimidyl)glycine

Benzyl [N - 1 - (S) - ethoxycarbonylethyl]alanyl - N - (2 - pyrimidyl)glycinate was dissolved in ethanol and 10% Pd/c was added under nitrogen. The reaction mixture was hydrogenated and purified to yield N - [1 - (S) - ethoxycarbonylethyl]alanyl - N - (2 - pyrimidyl)glycine.

By following the procedures in the above examples, the following additional compounds were prepared:

N-[1-(S)-Ethoxycarbonyl-2-phenylethyl]alanyl-N-(furfuryl)glycine
N-[1-(S)-Ethoxycarbonyl-3-methylbutyl]alanyl-N-(3-pyridyl)glycine
N-[1-(S)-Ethoxycarbonyl-3-phenylpropyl]alanyl-N-(tetrahydrofurfuryl)-glycine
N-[1-(S)-Ethoxycarbonyl-2-methylthioethyl]alanyl-N-[1-methyl-3-(2-indolylethyl)]-glycine
N-[1-Ethoxycarbonyl-4-methylpentyl]alanyl-N-(2-benzothiazole)alanine
N-[1-(S)-Ethoxycarbonyl-3-phenylpropyl]alanyl-N-(2-ethylmorpholine)glycine
N-[1-(S)-Ethoxycarbonyl-2-(3-indole)ethyl]alanyl-N-[(2-ethyl)pyrrolidine]glycine
N-[1-(S)-Ethoxycarbonylethyl]valyl-N-(5-indolyl)glycine
N-(1,3-Dicarboxypropyl)leucyl-N-(1,4-benzodioxan-6-yl)glycine
N-[1-(S)-Ethoxycarbonylethyl]isoleucyl-N-(5-benzofurfuryl)glycine
N-[1-(S)-Carboxyethyl]alanyl-N-(3-thienyl)glycine
N-[1-(S)-Carboxy-3-phenylpropyl]phenylalanyl-N-(3-thiazolyl)glycine
N-[1-(S)-Ethoxycarbonyl-2-phenylethyl]alanyl-N-(2-thienyl)glycine
N-(1,3-Diethoxycarbonylpropyl)-P-chlorophenylalanyl)-N′-(2-pyrimidyl)glycine
N-(1-(S)-Carboxy-3-methylbutyl)alanyl-N-(tetrahydrothiophene-1,1-dioxide-3yl)-lycine
N-(1-(S)-Ethoxycarbonyl-3-phenylpropyl)valyl-N-(N-ethylpiperidine-3-yl)-lycine
N-(1-(S)-Carboxy-2-phenylethyl)-phenylalanyl-N′-(4-tetrahydrothiopyranyl)-glycine.

The compounds of the present invention have demonstrated potent activity (of the order of $I_{50}$ of 1.0 to 50.0 micromols) in inhibiting the angiotensin converting enzyme (ACEI activity) when tested by the method described in Science *196*, 441—4 (1977). The compounds of the present invention have also demonstrated an $I_{50}$ of about 1 to 10 mg/kg P.O. in inhibiting infused angiotensin in rats. As such, these would be very useful in the treatment of hypertension.

The compounds may be administered orally or parenterally in the treatment of hypertension, and it will be within the professional judgement and skill of the practitioner to determine the amount to be administered. Suitable dosage forms include tablets, capsules, elixirs and injectables.

A particularly effective compound is N - (1 - carbethoxy - 2 - phenylethyl) - L - glycyl - N - [2 - (3 - indolylethyl)]glycine and related compounds including the indolylethyl substituent on the glycine nitrogen atom.

Example 13

The following example illustrates stereospecific synthesis:

A. Tert-butyl N-(3-pyridylinethyl) glycinate hydrochloride

Acetonitrile (300 ml) was added to 3-aminomethylpyridine (21.6 g, 0.2 mole) followed by the addition

of water (20 ml) and concentrated ammonium hydroxide (20 ml). To the resulting stirring solution *tert*-butyl bromoacetate (39 g, 0.2 mole) in acetonitrile (75 ml) was added dropwise at room temperature. The acetonitrile was evaporated on a rotary evaporator and then water was added to the residue and the product was extracted several times into methylene chloride. The combined methylene chloride extract was washed several times with water, dried over magnesium sulfate, filterd and concentrated to afford the crude product as a tan oil. The crude product was purified by silica-gel chromatography using methylene chloride as eluent. The desired product fractions were combined and concentrated to afford the desired product as an orange oil. The hydrochloride was prepared using anhydrous hydrogen chloride in ether to afford *tert*-butyl N-(3-methylpyridine)glycinate hydrochloride as a colorless powder (30 Fg, 59%); pr. P. 142°; mass spectra (CI): 223 (m+1, 100%).

*Anal.* calcd. for $C_{12}H_{18}N_2O_2$—Hcl: C, 55.70; H, 7.40; N, 10.83. Found: C, 55.11; H, 7.19; N, 10—50.

B. Tert - butyl N - [1 - (s) - ethoxy carbonyl - 3 - phenylpropyl] - (s) - alanyl - N - (3 - pyridylinethyl) glycinate hydrochloride

To N - [1 - (s) - ethoxy carbonyl - 3 - phenylpropyl - (S) - alanyl - N - carboxyanhydride (1.5 g, 4.92 mole) in methylene chloride (30 ml) was added *tert*-butyl N - (3 - pyridylmethyl)glycinate (1.4 g, 6.3 mole). The resulting solution was stirred overnight at room temperature. The solvent was evaporated and the residue was chromatographed over silica-gel using methylene chloride as eluent. The desired product fractions were combined and concentrated to give pure *tert*-butyl N - [1 - (S) - ethoxy carbonyl - 3 - phenylpropyl] - (S) - alanyl - N - (3 - methylpyridine)glycinate hydrochloride as a light colored oil (1.3 g, 54%). The hydrochloride of the product was prepared using anhydrous ether saturated with anhydrous hydrogen chloride to give a colorless solid which was filtered and washed with cold anhydrous ether: M.P. 76°; mass spectra (CI); 484.9 (m+1, 100%); [ ] $CHcl_3$=+50.99°; $[\ ]_{546}CHcl_3$=61.36°; $[\ ]_{436}CHcl_3$=109.64°; $[\ ]_{365}CHcl_3$=178.26°.

*Anal.* calcd. for $C_{27}H_{37}N_3O_5 \cdot 2Hcl$: C, 54.68; H, 6.29; N, 7.09. Found: C, 54.59; H, CHcl; N, 7.32.

C. N - [1 - (S) - Ethoxycarbonyl - 3 - phenylpropyl] - (S) - alanyl - N - (3 - pyridylinethyl)glycine dihydrochloride

To *tert*-butyl N - [1 - (S) - ethoxy carbonyl - 3 - phenylpropyl] - (S) - alanyl - N - (3 - pyridylinethyl)glycinate (.6 g, 1.24 mmoles) was added *P*-dioxane (30 ml) which had been saturated with anhydrous hydrogen chloride. The resulting solution was stirred for two-and-a-half hours at room temperature and then the solvent was evaporated to afford the desired product as a colorless powder (0.495 g, 86%): M.P. 77°; [ ] EtoH=+14.04°; mass spectra (CI): 410 (m+1—$H_2O$, 100%).

*Anal.* calcd. for $C_{23}H_{29}N_3O_5 \cdot 2Hcl—2H_2O$: C, 51.49; H, 6.58; N, 7.83. Found: C, 51.28; H, 6.86; N, 7.32.

**Claims for the Contracting States: BE, CH, DE, FR, GB, IT, LI, LU, NL, SE**

1. Compounds of the formula

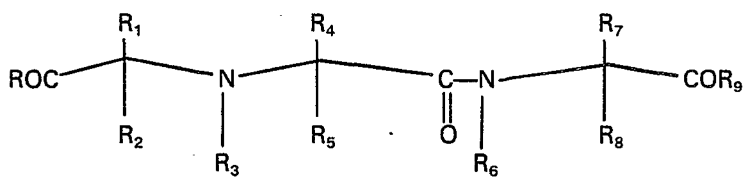

wherein

R and $R_9$ are independently hydroxy, lower alkoxy, lower alkenoxy, di(lower alkyl)amino-lower alkoxy, hydroxy-lower alkoxy, acylamino-lower alkoxy, acyloxy-lower alkoxy, aryloxy, aryloxyl-lower alkoxy, amino, lower alkylamino, di-lower alkylamino, hydroxyamino, or aryl-lower alkylamino,

$R_1$, $R_2$, $R_3$, $R_4$, $R_5$, $R_7$, and $R_8$ are independently hydrogen, alkyl, alkenyl or alkynyl containing up to 20 carbon atoms, aryl or aryl-lower alkyl containing up to 12 carbon atoms, heterocyclic or heterocyclic-lower alkyl containing up to 12 carbon atoms, or cycloalkyl or cycloalkyl alkyl containing up to 20 carbonations.

$R_6$ is heterocyclic or heterocyclic alkyl;

$R_2$ and $R_3$ taken together with the carbon and nitrogen to which they are respectively attached and $R_3$ and $R_5$ taken together with the nitrogen and carbon to which they are respectively attached form an N-heterocycle containing from 3 to 5 carbon atoms or 2 to 4 carbon atoms and a sulfur atom, and wherein said alkyl, alkenyl, and alkynyl groups can be substituted with hydroxy, lower alkoxy, thio, lower alkylmercapto, amino, lower alkylamino, di(lower alkyl)amino, halogen, and·nitro; and salts thereof, especially salts with pharmaceutically acceptable acids and bases.

2. A compound of the formula

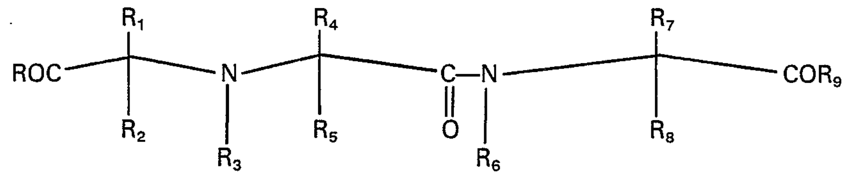

wherein:

R and $R_9$ are independently hydroxy and lower alkoxy,

$R_1$ and $R_2$ are independently hydrogen, lower alkyl, aryl-lower alkyl having from 7 to 12 carbon atoms, or heterocyclic-lower alkyl having from 6 to 12 carbon atoms,

$R_3$, $R_4$, $R_5$, $R_7$ and $R_8$ are hydrogen or lower alkyl, and

$R_6$ is heterocyclic or heterocyclic alkyl; and salts thereof with pharmaceutically-acceptable acids and bases.

3. Compound as in Claim 1 or Claim 2 wherein $R_6$ is pyrrole, pyrroline, pyrrolidine, pyridine, di-hydropyridine, piperidine, morpholine, thiamorpholine, imidazole, imidazoline, imidasolidine, furan, furfuryl, thiophene, benzimidazole, thiazole, thiazoline, thiazolidine, indole, quinoline, isoquinoline, tetrahydroquinoline, or tetrahydroisoquinoline.

4. Compound as in any of Claim 1—3 wherein the heterocyclic groups have 1 or more substituents selected from the group consisting of lower alkyl, lower alkenyl, lower alkynyl, hydroxy, lower alkoxy, amino, lower alkylamino, di(lower alkyl)amino, thiol, lower alkylmercapto, hydroxy-lower alkyl, amino-lower alkyl, thio-lower alkyl, nitro, halogen, trifluoromethyl, methylenedioxy, ureido or guanidino.

5. A compound as in any of Claims 1—4 wherein $R_1$ is lower alkyl or phenyl-lower alkyl, $R_4$ is lower alkyl, and $R_2$, $R_3$, $R_5$, $R_7$ and $R_8$ are hydrogen.

6. A compound as in any of Claims 1—4 wherein R and $R_9$ are hydroxy.

7. A compound as in any of Claims 1—4 wherein R is ethoxy and $R_9$ is hydroxy.

8. A compound as in any of Claims 1—7 wherein $R_1$ and $R_4$ are each methyl.

9. A compound as in any of Claims 1—7 wherein $R_1$ is benzyl or phenethyl and $R_4$ is methyl.

10. A compound as in any of Claims 1—9 wherein $R_6$ is indolylethyl.

11. A compound as in any of Claims 1—7 wherein $R_1$ is benzyl.

12. A compound as in any of Claims 1—7 wherein $R_1$ is phenethyl.

13. A compound as in Claims 1 or 2 wherein R is ethoxy, $R_1$ is phenethyl, $R_4$ is methyl, $R_6$ is 2 - (3 - indolylmethyl) and $R_9$ is hydroxy.

14. A compound as in Claim 13 wherein $R_2$, $R_3$, $R_5$, $R_7$ and $R_8$ are each hydrogen.

15. A compound as in Claims 1 or 2 wherein R is ethoxy; $R_1$ is phenethyl, $R_4$ is methyl, $R_6$ is 3-pyridylmethyl, morpholinyl, indolyl, pyrrolidinyl or indolealkyl, and $R_9$ is OH.

16. A compound as in Claim 15 wherein $R_2$, $R_3$, $R_5$, $R_7$ and $R_8$ are each hydrogen.

17. The process of preparing a compound of formula I herein which comprises reacting under amide-forming condition an amino compound of formula II with an acylating derivative of an acid of formula III; or reducing the corresponding imine; or by reaction to eliminate hydrogen halide of a compound of formula VI with a halo compound of formula VII; and optionally by substitution or conversion reactions introducing various substituents into the said products; and optionally forming salts thereof, especially pharmaceutically acceptable salts with acids and bases.

18. Process as in Claim 17 wherein

R and $R_9$ are independently hydroxy and lower alkoxy,

$R_1$ and $R_2$ are independently hydrogen, lower alkyl, aryl-lower alkyl having from 7 to 12 carbon atoms, or heterocyclic-lower alkyl having from 6 to 12 carbon atoms,

$R_3$, $R_4$, $R_5$, $R_7$ and $R_8$ are hydrogen or lower alkyl, and

$R_6$ is heterocyclic or heterocyclic alkyl; and salts thereof with pharmaceutically-acceptable acids and bases.

19. Process as in Claims 17 or 18 wherein $R_6$ is pyrrole, pyrroline, pyrrolidine, pyridine, di-hydropyridine, piperidine, morpholine, thiamorpholine, imidazole, imidazoline, imidazolidine, furan, furfuryl, thiophene, benzimidazole, thiazole, thiazoline, thiazolidine, indole, quinoline, isoquinoline, tetrahydroquinoline, or tetrahydroisoquinoline.

20. Process as in Claims 17—19 wherein the heterocyclic groups have 1 or more substituents selected from the group consisting of lower alkyl, lower alkenyl, lower alkynyl, hydroxy, lower alkoxy, amino, lower alkylamino, di(lower alkyl)amino, thiol, lower alkylmercapto, hydroxy-lower alkyl, amino-lower-alkyl, thio-lower alkyl, nitro, halogen, trifluoromethyl, methylenedioxy, ureido, or guanidino.

21. Process as in Claims 17—19 wherein $R_1$ is lower alkyl or phenyl-lower alkyl, $R_4$ is lower alkyl, and $R_2$, $R_3$, $R_5$, $R_7$ and $R_8$ are hydrogen.

22. Process as in any of Claims 17—19 wherein R and $R_9$ are hydroxy.

23. Process as in any of Claims 17—19 wherein R is ethoxy and $R_9$ is hydroxy.

24. Process as in any of Claims 17—19 wherein $R_1$ and $R_4$ are each methyl.

25. Process as in any of Claims 17—19 wherein $R_1$ is benzyl or phenethyl and $R_4$ is methyl.

26. Process as in any of Claims 17—19 wherein $R_6$ is indolyethyl.

27. Process as in any of Claims 17—19 wherein $R_1$ is benzyl.

28. Process as in any of Claims 17—19 wherein $R_1$ is phenethyl.

29. Process as in any of Claims 17—19 wherein R is ethoxy, $R_1$ is phenethyl, $R_4$ is methyl, $R_6$ is 2-(3-indolymethyl) and $R_9$ is hydroxy.

30. Process as in Claim 29 wherein $R_2$, $R_3$, $R_5$, $R_7$ and $R_8$ are each hydrogen.

31. A pharmaceutical composition containing at least one compound according to at least one of the claims 1 to 30.

**Claims for the Contracting State: AT**

1. A process for preparing compounds of the formula

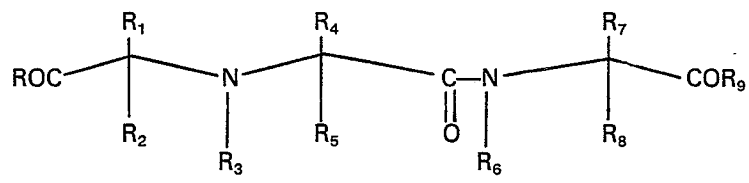

wherein

R and $R_9$ are independently hydroxy, lower alkoxy, lower alkenoxy, di(lower alkyl) amino-lower alkoxy, hydroxy-lower alkoxy, acylamino-lower alkoxy, acyloxy-lower alkoxy, aryloxy, aryloxyl-lower alkoxy, amino, lower alkylamino, di-lower alkylamino, hydroxyamino, or aryl-lower alkylamino,

$R_1$, $R_2$, $R_3$, $R_4$, $R_5$, $R_7$ and $R_8$ are independently hydrogen, alkyl, alkenyl or alkynyl containing up to 20 carbon atoms, aryl or aryl-lower alkyl containing up to 12 carbon atoms, heterocyclic or heterocyclic-lower alkyl containing up to 12 carbon atoms, or cycloalkyl or cycloalkyl alkyl containing up to 20 carbon atoms,

$R_6$ is heterocyclic or heterocyclic alkyl;

$R_2$ and $R_3$ taken together with the carbon and nitrogen to which they are respectively attached and $R_3$ and $R_5$ taken together with the nitrogen and carbon to which they are respectively attached form an N-heterocycle containing from 3 to 5 carbon atoms or 2 to 4 carbon atoms and a sulfur atom, and wherein said alkyl, alkenyl and alkynyl groups can be substituted with hydroxy, lower alkoxy, thio, lower alkylmercapto, amino, lower alkylamino, di(lower alkyl)amino, halogen and nitro; and salts thereof, especially pharmaceutically acceptable salts with acids or bases, which comprises reacting under amide-forming condition an amino compound of Formula II

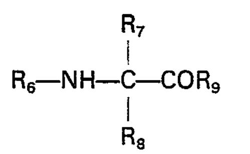

(II)

with an acylating derivative of an acid of Formula III;

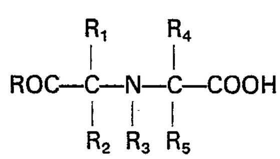

(III)

or reducing the corresponding imine; or by reaction to eliminate hydrogen halide of a compound of Formula VI

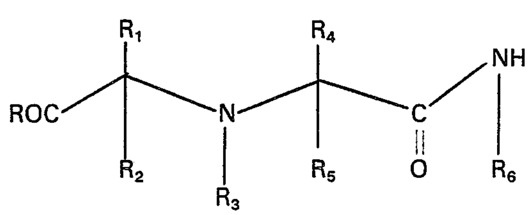

(VI)

with a halo compound of Formula VII:

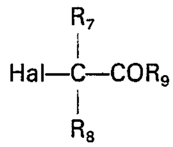
(VII)

and optionally by substitution or conversion reactions introducing various substituents into the said products; and optionally forming salts thereof, especially pharmaceutically acceptable salts with acids and bases.

2. The process of Claim 1 wherein R and $R_9$ are independently hydroxy and lower alkoxy,

$R_1$ and $R_2$ are independently hydrogen, lower alkyl, aryl-lower alkyl having from 7 to 12 carbon atoms, or heterocyclic-lower alkyl having from 6 to 12 carbon atoms,

$R_3$, $R_4$, $R_5$, $R_7$ and $R_8$ are hydrogen or lower alkyl, and

$R_6$ is heterocyclic or heterocyclic alkyl; and salts thereof with pharmaceutically-acceptable acids and bases.

3. The process of Claims 1 or 2 wherein $R_6$ is pyrrole, pyrroline, pyrrolidine, pyridine, di-hydropyridine, piperidine, morpholine, thiamorpholine, imidazole, imidazoline, imidazolidine, furan, furfuryl, thiophene, benzimidazole, thiazole, thiazoline, thiazolidine, indole, quinoline, isoquinoline, tetrahydroquinoline, or tetrahydroisoquinoline.

4. The process of any of Claims 1 to 3 wherein the heterocyclic groups have 1 or more substituents selected from the group consisting of lower alkyl, lower alkenyl, lower alkynyl, hydroxy, lower alkoxy, amino, lower alkylamino, di(lower alkyl)amino, thiol, lower alkylmercapto, hydroxy-lower alkyl, amino-lower-alkyl, thio-lower alkyl, nitro, halogen, trifluoromethyl, methylenedioxy, ureido, or guanidino.

5. The process of any of Claims 1 to 4 wherein $R_1$ is lower alkyl or phenyl-lower alkyl, $R_4$ is lower alkyl and $R_2$, $R_3$, $R_5$, $R_7$ and $R_8$ are hydrogen.

6. The process of any of Claims 1 to 4 wherein R and $R_9$ are hydroxy.

7. The process of any of claims 1 to 4 wherein R is ethoxy and $R_9$ is hydroxy.

8. The process of any of Claims 1 to 7 wherein $R_1$ and $R_4$ are each methyl.

9. The process of any of Claims 1 to 7 wherein $R_1$ is benzyl or phenethyl and $R_4$ is methyl.

10. The process of any of Claims 1 to 7 wherein $R_6$ is indolylethyl.

11. The process of any of Claims 1 to 7 wherein $R_1$ is benzyl.

12. The process of any of Claims 1 to 7 wherein $R_1$ is phenethyl.

13. The process of any of Claims 1 to 4 wherein R is ethoxy, $R_1$ is phenethyl, $R_4$ is methyl, $R_6$ is 2-(3-indolylmethyl) and $R_9$ is hydroxy.

14. The process of Claim 13 wherein $R_2$, $R_3$, $R_5$, $R_7$ and $R_8$ are each hydrogen.

15. The process of Claims 1 or 2 wherein R is ethoxy; $R_1$ is phenethyl, $R_4$ is methyl, $R_6$ is 3-pyridylmethyl, morpholinyl, indolyl, pyrrolidinyl or indolealkyl and $R_9$ is OH.

16. The process of Claim 15 wherein $R_2$, $R_3$, $R_5$, $R_7$ and $R_8$ are each hydrogen.

17. A process for preparing a pharmaceutical composition comprising formulating at least one compound of the formula according to Claim 1 together with usual pharmaceutical carriers.

**Patentansprüche für die Vertragsstaaten: BE, CH, DE, FR, GB, IT, LI, LU, NL, SE**

1. Verbindungen der Formel

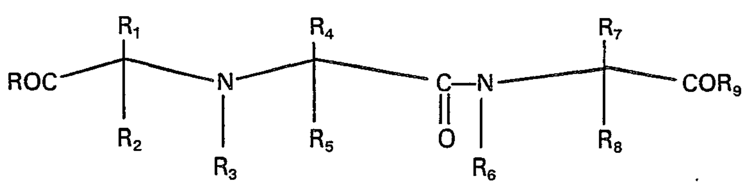

worin

R und $R_9$ unabhängig voneinander Hydroxy, Niedrigalkoxy, Niedrigalkenoxy, Di-(niedrigalkyl)-amino-niedrigalkoxy, Hydroxy-niedrigalkoxy, Acylamino-niedrigalkoxy, Acyloxy-niedrigalkoxy, Aryloxy, Aryloxy-niedrigalkoxy, Amino, Niedrigalkylamino, Di-niedrigalkylamino, Hydroxyamino oder Aryl-niedrigalkylamino bedeuten,

$R_1$, $R_2$, $R_3$, $R_4$, $R_5$, $R_7$ und $R_8$ unabhängig voneinander Wasserstoff, Alkyl, Alkenyl oder Alkynyl mit bis zu 20 Kohlenstoffatomen, Aryl oder Aryl-niedrigalkyl mit bis zu 12 Kohlenstoffatomen, Heterocyclo oder Heterocyclo-niedrigalkyl mit bis zu 12 Kohlenstoffatomen oder Cycloalkyl oder Cycloalkylalkyl mit bis zu 20 Kohlenstoffatomen bedeuten;

$R_6$ Heterocyclo oder Heterocyclo-alkyl bedeutet;

$R_2$ und $R_3$ zusammen mit dem Kohlenstoff und Stickstoff, an den sie jeweils gebunden sind, und $R_3$ und $R_5$ zusammen mit dem Stickstoff und Kohlenstoff, an den sie jeweils gebunden sind, einen N-Heterocyclus mit 3 bis 5 Kohlenstoffatomen oder 2 bis 4 Kohlenstoffatomen und einem Schwefelatom bilden, und worin

die Alkyl-, Alkenyl- und Alkynylgruppen durch Hydroxy, Niedrigalkoxy, Thio, Niedrigalkylmercapto, Amino, Niedrigalkylamino, Di-(niedrigalkyl)-amino, Halogen und Nitro substituiert sein können, und Salze davon, insbesondere Salze mit pharmazeutisch annehmbaren Säuren und Basen.

2. Verbindung der Formel

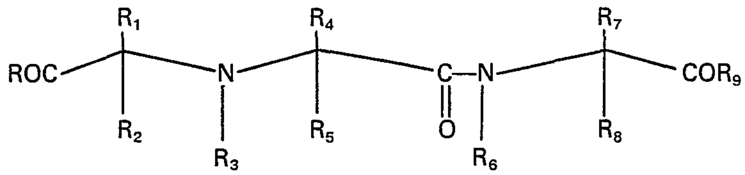

worin

R und $R_9$ unabhängig voneinander Hydroxy und Niedrigalkoxy bedeuten,

$R_1$ und $R_2$ unabhängig voneinander Wasserstoff, Niedrigalkyl, Aryl-niedrigalkyl mit 7 bis 12 Kohlenstoffatomen oder Heterocyclo-niedrigalkyl mit 6 bis 12 Kohlenstoffatomen bedeuten,

$R_3$, $R_4$, $R_5$, $R_7$ und $R_8$ Wasserstoff oder Niedrigalkyl bedeuten und

$R_6$ Heterocyclo oder Heterocyclo-alkyl bedeutet; und Salze davon mit pharmazeutisch annehmbaren Säuren und Basen.

3. Verbindung nach Anspruch 1 oder 2, worin $R_6$ Pyrrol, Pyrrolin, Pyrrolidin, Pyridin, Di-hydropyridin, Piperidin, Morpholin, Thiamorpholin, Imidazol, Imidazolin, Imidazolidin, Furan, Furfuryl, Thiophen, Benzimidazol, Thiazol, Thiazolin, Thiazolidin, Indol, Chinolin, Isochinolin, Tetrahydrochinolin oder Tetrahydroisochinolin bedeutet.

4. Verbindung nach einem der Ansprüche 1 bis 3, worin die heterocyclischen Gruppen einen oder mehrere Substituenten, gewählt aus der Gruppe, bestehend aus Niedrigalkyl, Niedrigalkenyl, Niedrigalkynyl, Hydroxy, Niedrigalkoxy, Amino, Niedrigalkylamino, Di-(niedrigalkyl)-amino, Thiol, Niedrigalkylmercapto, Hydroxyniedrigalkyl, Amino-niedrigalkyl, Thio-niedrigalkyl, Nitro, Halogen, Trifluormethyl, Methylendioxy, Ureido oder Guanidino, besitzen.

5. Verbindung nach einem der Ansprüche 1 bis 4, worin $R_1$ Niedrigalkyl oder Phenylniedrigalkyl bedeutet, $R_4$ Niedrigalkyl bedeutet und $R_2$, $R_3$, $R_5$, $R_7$ und $R_8$ Wasserstoff bedeuten.

6. Verbindung nach einem der Ansprüche 1 bis 4, worin R und $R_9$ Hydroxy bedeuten.

7. Verbindung nach einem der Ansprüche 1 bis 4, worin R Ethoxy und $R_9$ Hydroxy bedeuten.

8. Verbindung nach einem der Ansprüche 1 bis 7, worin $R_1$ und $R_4$ jeweils Methyl bedeuten.

9. Verbindung nach einem der Ansprüche 1 bis 7, worin $R_1$ Benzyl oder Phenethyl bedeutet und $R_4$ Methyl bedeutet.

10. Verbindung nach einem der Ansprüche 1 bis 9, worin $R_6$ Indolylethyl bedeutet.

11. Verbindung nach einem der Ansprüche 1 bis 7, worin $R_1$ Benzyl bedeutet.

12. Verbindung nach einem der Ansprüche 1 bis 7, worin $R_1$ Phenethyl bedeutet.

13. Verbindung nach Anspruch 1 oder 2, worin R Ethoxy, $R_1$ Phenethyl, $R_4$ Methyl, $R_6$ 2-(3-Indolylmethyl) und $R_9$ Hydroxy bedeutet.

14. Verbindung nach Anspruch 13, worin $R_2$, $R_3$, $R_5$, $R_7$ und $R_8$ jeweils Wasserstoff bedeuten.

15. Verbindung nach Anspruch 1 oder 2, worin R Ethoxy, $R_1$ Phenethyl, $R_4$ Methyl, $R_6$ 3-Pyridylmethyl, Morpholinyl, Indolyl, Pyrrolidinyl oder Indolalkyl und $R_9OH$ bedeutet.

16. Verbindung nach Anspruch 15, worin $R_2$, $R_3$, $R_5$, $R_7$ und $R_8$ jeweils Wasserstoff bedeuten.

17. Verfahren zur Herstellung einer Verbindung der Formel I, welches die Umsetzung einer Aminoverbindung der Formel II mit einem acylierenden Derivat einer Säure der Formel III unter amidbildender Bedingung, oder die Reduktion des entsprechenden Imins, oder die Umsetzung einer Verbindung der Formel IV mit einer Halogenverbindung der Formel VII zur Abspaltung von Halogenwasserstoff und gegebenenfalls die Einführung verschiedener Substituenten in die Produkte durch Substitutions- oder Umwandlungsreaktionen und gegebenenfalls die Bildung von Salzen davon, insbesondere pharmazeutisch annehmbaren Salzen mit Säuren und Basen, umfaßt.

18. Verfahren nach Anspruch 17, worin R und $R_9$ unabhängig voneinander Hydroxy und Niedrigalkoxy bedeuten,

$R_1$ und $R_2$ unabhängig voneinander Wasserstoff, Niedrigalkyl, Aryl-niedrigalkyl mit 7 bis 12 Kohlenstoffatomen oder Heterocyclo-niedrigalkyl mit 6 bis 12 Kohlenstoffatomen bedeuten,

$R_3$, $R_4$, $R_5$, $R_7$ und $R_8$ Wasserstoff oder Niedrigalkyl bedeuten und

$R_6$ Heterocyclo oder Heterocyclo-alkyl bedeutet; und Salze davon mit pharmazeutisch annehmbaren Säuren und Basen.

19. Verfahren nach Anspruch 17 oder 18, worin $R_6$ Pyrrol, 1 Pyrrolin, Pyrrolidin, Pyridin, Dihydropyridin, Piperidin, Morpholin, Thiamorpholin, Imidazol, Imidazolin, Imidazolidin, Furan, Furfuryl, Thiophen, Benzimidazol, Thiazol, Thiazolin, Thiazolidin, Indol, Chinolin, Isochinolin, Tetrahydrochinolin oder Tetrahydroisochinolin bedeutet.

20. Verfahren nach einem der Ansprüche 17 bis 19, worin die heterocyclischen Gruppen einen oder mehrere Substituenten, gewählt aus der Gruppe, bestehend aus Niedrigalkyl, Niedrigalkenyl, Niedrigalkynyl, Hydroxy, Niedrigalkoxy, Amino, Niedrigalkylamino, Di-(niedrigalkyl)-amino, Thiol,

Niedrigalkylmercapto, Hydroxyniedrigalkyl, Amino-niedrigalkyl, Thio-niedrigalkyl, Nitro, Halogen, Trifluormethyl, Methylendioxy, Ureido oder Guanidino, besitzen.

21. Verfahren nach einem der Ansprüche 17 bis 19, worin $R_1$ Niedrigalkyl oder Phenylniedrigalkyl bedeutet, $R_4$ Niedrigalkyl bedeutet und $R_2$, $R_3$, $R_5$, $R_7$ und $R_8$ Wasserstoff bedeuten.

22. Verfahren nach einem der Ansprüche 17 bis 19, worin R und $R_9$ Hydroxy bedeuten.

23. Verfahren nach einem der Ansprüche 17 bis 19, worin R Ethoxy und $R_9$ Hydroxy bedeuten.

24. Verfahren nach einem der Ansprüche 17 bis 19, worin $R_1$ und $R_4$ jeweils Methyl bedeuten.

25. Verfahren nach einem der Ansprüche 17 bis 19, worin $R_1$ Benzyl oder Phenethyl bedeutet und $R_4$ Methyl bedeutet.

26. Verfahren nach einem der Ansprüche 17 bis 19, worin $R_6$ Indolylethyl bedeutet.

27. Verfahren nach einem der Ansprüche 17 bis 19, worin $R_1$ Benzyl bedeutet.

28. Verfahren nach einem der Ansprüche 17 bis 19, worin $R_1$ Phenethyl bedeutet.

29. Verfahren nach einem der Ansprüche 17 bis 19, worin R Ethoxy, $R_1$ Phenethyl, $R_4$ Methyl, $R_6$ 2-(3-Indolylmethyl) und $R_9$ Hydroxy bedeutet.

30. Verfahren nach Anspruch 29, worin $R_2$, $R_3$, $R_5$, $R_7$ und $R_8$ jeweils Wasserstoff bedeuten.

31. Pharmazeutische Zusammensetzung, enthaltend wenigstens eine Verbindung nach wenigstens einem der Ansprüche 1 bis 30.

**Patentansprüche für den Vertragsstaat: AT**

1. Verfahren zur Herstellung von Verbindungen der Formel

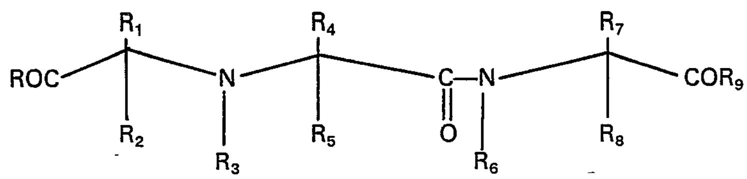

worin

R und $R_9$ unabhängig voneinander Hydroxy, Niedrigalkoxy, Niedrigalkenoxy, Di-(niedrigalkyl)-amino-niedrigalkoxy, Hydroxy-niedrigalkoxy, Acylamino-niedrigalkoxy, Acyloxy-niedrigalkoxy, Aryloxy, Aryloxy-niedrigalkoxy, Amino, Niedrigalkylamino, Di-niedrigalkylamino, Hydroxyamino oder Aryl-niedrigalkylamino bedeuten,

$R_1$, $R_2$, $R_3$, $R_4$, $R_5$, $R_7$ und $R_8$ unabhängig voneinander Wasserstoff, Alkyl, Alkenyl oder Alkynyl mit bis zu 20 Kohlenstoffatomen, Aryl oder Aryl-niedrigalkyl mit bis zu 12 Kohlenstoffatomen, Heterocyclo oder Heterocyclo-niedrigalkyl mit bis zu 12 Kohlenstoffatomen oder Cycloalkyl oder Cycloalkylalkyl mit bis zu 20 Kohlenstoffatomen bedeuten,

$R_6$ Heterocyclo oder Heterocyclo-alkyl bedeutet;

$R_2$ und $R_3$ zusammen mit dem Kohlenstoff und Stickstoff, an den sie jeweils gebunden sind, und $R_3$ und $R_5$ zusammen mit dem Stickstoff und Kohlenstoff, an den sie jeweils gebunden sind, einen N-Heterocyclus mit 3 bis 5 Kohlenstoffatomen oder 2 bis 4 Kohlenstoffatomen und einem Schwefelatom bilden, und worin die Alkyl-, Alkenyl- und Alkynylgruppen durch Hydroxy, Niedrigalkoxy, Thio, Niedrigalkylmercapto, Amino, Niedrigalkylamino, Di-(niedrigalkyl)-amino, Halogen und Nitro substituiert sein können, und Salze davon, insbesondere Salze mit pharmazeutisch annehmbaren Säuren und Basen, welches die Umsetzung einer Aminoverbindung der Formel II

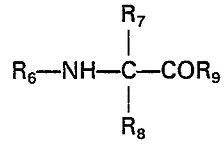

(II)

mit einem acylierenden Derivat einer Säure der Formel III

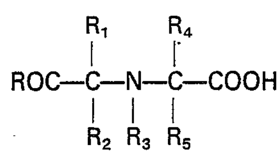

(III)

unter amidbildender Bedingung, oder die Reduktion des entsprechenden Imins, oder die Umsetzung einer Verbindung der Formel VI

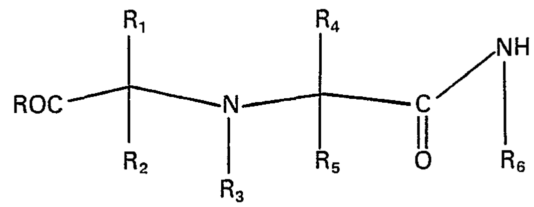

(VI)

mit einerm Halogenverbindung der Formel VII

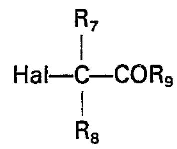

(VII)

zur Abspaltung von Halogenwasserstoff, und gegebenenfalls die Einführung verschiedener Substituenten in die Produkte durch Substitutions- oder Umwandlungsreaktionen und gegebenenfalls die Bildung von Salzen davon, insbesonders pharmazeutisch annehmbarer Salze mit Säuren und Basen, umfaßt.

2. Verfahren nach Anspruch 1, worin R und $R_9$ unabhängig voneinander Hydroxy und Niedrigalkoxy bedeuten,

$R_1$ und $R_2$ unabhängig voneinander Wasserstoff, Niedrigalkyl, Aryl-niedrigalkyl mit 7 bis 12 Kohlenstoffatomen oder Heterocyclo-niedrigalkyl mit 6 bis 12 Kohlenstoffatomen bedeuten,

$R_3$, $R_4$, $R_5$, $R_7$ und $R_8$ Wasserstoff oder Niedrigalkyl bedeuten und

$R_6$ Heterocyclo oder Heterocyclo-alkyl bedeutet; und Salze davon mit pharmazeutisch annehmbaren Säuren und Basen.

3. Verfahren nach Anspruch 1 oder 2, worin $R_6$ Pyrrol, Pyrrolin, Pyrrolidin, Pyridin, Dihydropyridin, Piperidin, Morpholin, Thiamorpholin, Imidazol, Imidazolin, Imidazolidin, Furan, Furfuryl, Thiophen, Benzimidazol, Thiazol, Thiazolin, Thiazolidin, Indol, Chinolin, Isochinolin, Tetrahydrochinolin oder Tetrahydroisochinolin bedeutet.

4. Verfahren nach einem der Ansprüche 1 bis 3, worin die heterocyclischen Gruppen einen oder mehrere Substituenten, gewählt aus der Gruppe, bestehend aus Niedrigalkyl, Niedrigalkenyl, Niedrigalkynyl, Hydroxy, Niedrigalkoxy, Amino, Niedrigalkylamino, Di-(niedrigalkyl)-amino, Thiol, Niedrigalkylmercapto, Hydroxyniedrigalkyl, Amino-niedrigalkyl, Thio-niedrigalkyl, Nitro, Halogen, Trifluormethyl, Methylendioxy, Ureido oder Guanidino, besitzen.

5. Verfahren nach einem der Ansprüche 1 bis 4, worin $R_1$ Niedrigalkyl oder Phenylniedrigalkyl bedeutet, $R_4$ Niedrigalkyl bedeutet und $R_2$, $R_3$, $R_5$, $R_7$ und $R_8$ Wasserstoff bedeuten.

6. Verfahren nach einem der Ansprüche 1 bis 4, worin R und $R_9$ Hydroxy bedeuten.

7. Verfahren nach einem der Ansprüche 1 bis 4, worin R Ethoxy und $R_9$ Hydroxy bedeuten.

8. Verfahren nach einem der Ansprüche 1 bis 7, worin $R_1$ und $R_4$ jeweils Methyl bedeuten.

9. Verfahren nach einem der Ansprüche 1 bis 7, worin $R_1$ Benzyl oder Phenethyl bedeutet und $R_4$ Methyl bedeutet.

10. Verfahren nach einem der Ansprüche 1 bis 7, worin $R_6$ Indolylethyl bedeutet.

11. Verfahren nach einem der Ansprüche 1 bis 7, worin $R_1$ Benzyl bedeutet.

12. Verfahren nach einem der Ansprüche 1 bis 7, worin $R_1$ Phenethyl bedeutet.

13. Verfahren nach einem der Ansprüche 1 bis 4, worin R Ethoxy, $R_1$ Phenethyl, $R_4$ Methyl, $R_6$ 2-(3-Indolylmethyl) und $R_9$ Hydroxy bedeutet.

14. Verfahren nach Anspruch 13, worin $R_2$, $R_3$, $R_5$, $R_7$ und $R_8$ jeweils Wasserstoff bedeuten.

15. Verfahren nach einem der Ansprüche 1 oder 2, worin R Ethoxy, $R_1$ Phenethyl, $R_4$ Methyl, $R_6$ 3-Pyridylmethyl, Morpholinyl, Indolyl, Pyrrolidinyl oder Indolalkyl und $R_9$ OH bedeutet.

16. Verfahren nach Anspruch 15, worin $R_2$, $R_3$, $R_5$, $R_7$ und $R_8$ jeweils Wasserstoff bedeuten.

17. Verfahren zur Herstellung einer pharmazeutischen Zusammensetzung, welches die Formulierung wenigstens einer Verbindung der Formel gemäß Anspruch 1 zusammen mit üblichen pharmazeutischen Trägern umfaßt.

**Revendications pour les Etats Contractants: BE, DE, CH, LI, FR, GB, IT, LU, NL, SE**

1. Composé de la formule:

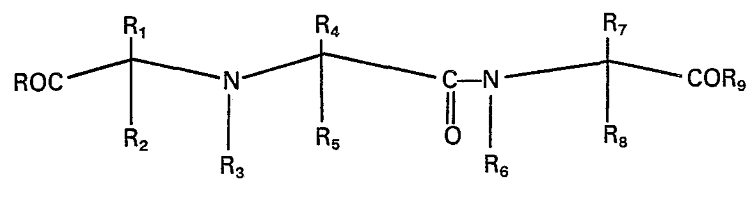

dans laquelle

R et $R_9$ sont indépendamment des groupes hydroxyle, alcoxyle inférieur, alcénoxyle inférieur, bis-(alkyle inférieur)-amino-alcoxyle inférieur, hydroxy-alcoxyle inférieur, acylamino-alcoxyle inférieur, acyloxy-alcoxyle inférieur, aryloxyle, aryloxy-alcoxyle inférieur, amine, alkylamine inférieur, bis-(alkyle inférieur)-amine, hydroxyamine ou aryl-alkylamine inférieur,

$R_1$, $R_2$, $R_3$, $R_4$, $R_5$, $R_7$ et $R_8$ sont indépendamment des atomes d'hydrogène, des groupes alkyle, alcényle ou alcynyle contenant jusqu'à 20 atomes de carbone, aryle ou aryl-alkyle inférieur contenant jusqu'à 12 atomes de carbone, hétérocycliques ou hétérocyclique-alkyle inférieur contenant jusqu'à 12 atomes de carbone, ou cycloalkyle ou cycloalkyle-alkyle contenant jusqu'à 20 atomes de carbone,

$R_6$ est un groupe hétérocyclique ou hétérocyclique-alkyle,

$R_2$ et $R_3$, pris ensemble avec les atomes de carbone et d'azote auxquels ils sont respectivement rattachés, et $R_3$ et $R_5$, pris ensemble avec les atomes d'azote et de carbone auxquels ils sont respectivement rattachés, forment un hétérocycle azoté contenant de 3 à 5 atomes de carbone ou 2 à 4 atomes de carbone et un atome de soufre, et dans laquelle lesdits groupes alkyle, alcényle et alcynyle peuvent être substitués par des groupes hydroxyle, alcoxyle inférieur, thio, alkyl inférieurmercapto, amine, alkyle inférieur-amine, bis-(alkyle inférieur)-amine, des halogènes et des groupes nitro, et leurs sels, spécialement leurs sels d'acides et bases pharmaceutiquement acceptables.

2. Composé de la formule:

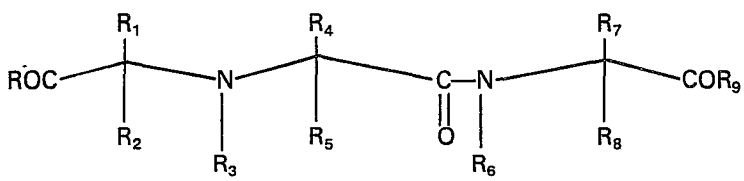

dans laquelle:

R et $R_9$ sont indépendamment des groupes hydroxyle et alcoxyle inférieur,

$R_1$ et $R_2$ sont indépendamment des atomes d'hydrogène, des groupes alkyle inférieur, aryl-alkyle inférieur ayant de 7 à 12 atomes de carbone, ou hétérocyclique-alkyle inférieur ayant de 6 à 12 atomes de carbone,

$R_3$, $R_4$, $R_5$, $R_7$ et $R_8$ sont des atomes d'hydrogène ou des groupes alkyle inférieur,

$R_6$ est un groupe hétérocyclique ou hétérocyclique-alkyle; et ses sels d'acides et bases pharmaceutiquement acceptables.

3. Composé selon l'une des revendications 1 et 2, dans lequel $R_6$ est un groupe pyrrole, pyrroline, pyrrolidine, pyridine, dihydropyridine, pipéridine, morpholine, thiamorpholine, imidazole, imidazoline, imidazolidine, furanne, furfuryle, thiophène, benzimidazole, thiazole, thiazoline, thiazolidine, indole, quinoléine, isoquinoléine, tétrahydroquinoléine ou tétrahydroisoquinoléine.

4. Composé selon l'une quelconque des revendications 1 à 3, dans lequel les groupes hétérocycliques ont un ou plusieurs substituants choisis dans le groupe formé des groupes alkyle inférieur, alcényle inférieur, alcynyle inférieur, hydroxyle, alcoxyle inférieur, amine, alkyle inférieur-amine, bis-(alkyle inférieur)-amine, thiol, alkyle inférieur-mercapto, hydroxyalkyle inférieur, aminoalkyle inférieur, thioalkyle inférieur, nitro, les halogènes, les groupes trifluoro-méthyle, méthylènedioxy, uréido ou guanidino.

5. Composé selon l'une quelconque des revendications 1 à 4, dans lequel $R_1$ est un groupe alkyle inférieur ou phényl-alkyle inférieur, $R_4$ est un groupe alkyle inférieur et $R_2$, $R_3$, $R_5$, $R_7$ et $R_8$ sont des atomes d'hydrogène.

6. Composé selon l'une quelconque des revendications 1 à 4, dans lequel R et $R_9$ sont des groupes hydroxyle.

7. Composé selon l'une quelconque des revendications 1 à 4, dans lequel R est un groupe éthoxyle et $R_9$ est un groupe hydroxyle.

8. Composé selon l'une quelconque des revendications 1 à 7, dans lequel $R_1$ et $R_4$ sont chacun un groupe méthyle.

9. Composé selon l'une quelconque des revendications 1 à 7, dans lequel $R_1$ est un groupe benzyle ou phénéthyle et $R_4$ est un groupe méthyle.

10. Composé selon l'une quelconque des revendications 1 à 9, dans lequel $R_6$ est un groupe indolyléthyle.

11. Composé selon l'une quelconque des revendications 1 à 7, dans lequel $R_1$ est un groupe benzyle.

12. Composé selon l'une quelconque des revendications 1 à 7, dans lequel $R_1$ est un groupe phénéthyle.

13. Composé selon l'une des revendications 1 et 2, dans lequel R est un groupe éthoxyle, $R_1$ est un groupe phénéthyle, $R_4$ est un groupe méthyle, $R_6$ est un groupe 2-(3-indolylméthyle) et $R_9$ est un groupe hydroxyle.

14. Composé selon la revendication 13, dans lequel $R_2$, $R_3$, $R_5$, $R_7$ et $R_8$ sont chacun un atome d'hydrogène.

15. Composé selon l'une des revendications 1 et 2, dans lequel R est un groupe éthoxyle; $R_1$ est un

groupe phénéthyle, $R_4$ est un groupe méthyle, $R_6$ est un groupe 3-pyridylméthyle, morpholinyle, indolyle, pyrrolidinyle ou indole-alkyle et $R_9$ est OH.

16. Composé selon la revendication 15, dans lequel $R_2$, $R_3$, $R_5$, $R_7$ et $R_8$ sont chacun un atome d'hydrogène.

17. Procédé de préparation d'un composé de la formule I ici indiquée, qui consiste à faire réagir, dans des conditions de formation d'amide, un composé aminé de formule II sur un dérivé acylant d'un acide de formule III; ou à réduire l'imine correspondante; ou par réaction, pour éliminer de l'halogénure d'hydrogène, d'un composé de formule VI sur un composé halogéné de formule VII; et facultativement, par des réactions de substitution ou de conversion à introduire divers substituants dans les produits; et facultativement, à en former des sels, spécialement des sels d'acides et bases pharmaceutiquement acceptables.

18. Procédé selon la revendication 17, dans lequel R et $R_9$ sont indépendamment des groupes hydroxyle et alcoxyle inférieur,

$R_1$ et $R_2$ sont indépendamment des atomes d'hydrogène, des groupes alkyle inférieur, aryl-alkyle inférieur ayant de 7 à 12 atomes de carbone ou hétérocyclique-alkyle inférieur ayant de 6 à 12 atomes de carbone,

$R_3$, $R_4$, $R_5$, $R_7$ et $R_8$ sont des atomes d'hydrogène ou des groupes alkyle inférieur, et

$R_6$ est un groupe hétérocyclique ou hétérocyclique-alkyle; et leurs sels d'acides et bases pharmaceutiquement acceptables.

19. Procédé selon l'une des revendications 17 et 18, dans lequel $R_6$ est un groupe pyrrole, pyrroline, pyrrolidine, pyridine, dihydropyridine, pipéridine, morpholine, thiamorpholine, imidazole, imidazoline, imidazolidine, furanne, furfuryle, thiophène, benzimidazole, thiazole, thiazoline, thiazolidine, indole, quinoléine, isoquinoléine, tétrahydroquinoléine ou tétrahydroisoquinoléine.

20. Procédé selon les revendications 17 à 19, dans lequel les groupes hétérocycliques ont un ou plusieurs substituants choisis dans le groupe formé des groupes alkyle inférieur, alcényle inférieur, alcynyle inférieur, hydroxyle, alcoxyle inférieur, amine, alkylamine inférieur, bis-(alkyle inférieur)-amine, thiol, alkyle inférieur-mercapto, hydroxyalkyle inférieur, aminoalkyle inférieur, thioalkyle inférieur, nitro, les halogènes, les groupes trifluorométhyle, méthylènedioxy, uréido ou guanidino.

21. Procédé selon les revendications 17 à 19, dans lequel $R_1$ est un groupe alkyle inférieur ou phénylalkyle inférieur, $R_4$ est un groupe alkyle inférieur et $R_2$, $R_3$, $R_5$, $R_7$ et $R_8$ sont des atomes d'hydrogène.

22. Procédé selon l'une quelconque des revendications 17 à 19, dans lequel R et $R_9$ sont des groupes hydroxyle.

23. Procédé selon l'une quelconque des revendications 17 à 19, dans lequel R est un groupe éthoxyle et $R_9$ est un groupe hydroxyle.

24. Procédé selon l'une quelconque des revendications 17 à 19, dans lequel $R_1$ et $R_4$ sont chacun un groupe méthyle.

25. Procédé selon l'une quelconque des revendications 17 à 19, dans lequel $R_1$ est un groupe benzyle ou phénéthyle et $R_4$ est un groupe méthyle.

26. Procédé selon l'une quelconque des revendications 17 à 19, dans lequel $R_6$ est un groupe indolyléthyle.

27. Procédé selon l'une quelconque des revendications 17 à 19, dans lequel $R_1$ est un groupe benzyle.

28. Procédé selon l'une quelconque des revendications 17 à 19, dans lequel $R_1$ est un groupe phénéthyle.

29. Procédé selon l'une quelconque des revendications 17 à 19, dans lequel R est un groupe éthoxyle, $R_1$ est un groupe phénéthyle, $R_4$ est un groupe méthyle, $R_6$ est un groupe 2-(3-indolylméthyle) et $R_9$ est un groupe hydroxyle.

30. Procédé selon la revendication 29, dans lequel $R_2$, $R_3$, $R_5$, $R_7$ et $R_8$ sont chacun un atome d'hydrogène.

31. Composition pharmaceutique contenant au moins un composé selon au moins une des revendications 1 à 30.

**Revendications pour l'Etat Contractant: AT**

1. Procédé de préparation de composés de la formule:

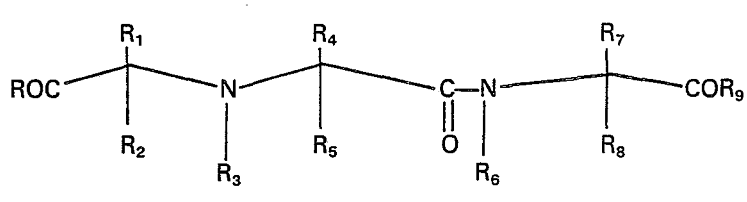

dans laquelle

R et $R_9$ sont indépendamment des groupes hydroxyle, alcoxyle inférieur, alcénoxyle, inférieur, bis-(alkyle inférieur)-amino-alcoxyle inférieur, hydroxy-alcoxyle inférieur, acylamino-alcoxyle inférieur,

acyloxy-alcoxyle inférieur, aryloxyle, aryloxy-alcoxyle inférieur, amine, alkylamine inférieur, bis-(alkyle inférieur)-amine, hydroxyamine ou aryl-alkylamine inférieur,

$R_1$, $R_2$, $R_3$, $R_4$, $R_5$, $R_7$ et $R_8$ sont indépendamment des atomes d'hydrogène, des groupes alkyle, alcényle ou alcynyle contenant jusqu'à 20 atomes de carbone, aryle ou aryl-alkyle inférieur contenant jusqu'à 12 atomes de carbone, hétérocycliques ou hétérocyclique-alkyle inférieur contenant jusqu'à 12 atomes de carbone, ou cycloalkyle ou cycloalkylalkyle contenant jusqu'à 20 atomes de carbone,

$R_6$ est un groupe hétérocyclique ou hétérocyclique-alkyle,

$R_2$ et $R_3$, pris ensemble avec les atomes de carbone et d'azote auxquels ils sont respectivement rattachés, et $R_3$ et $R_5$, pris ensemble avec les atomes d'azote et de carbone auxquels ils sont respectivement rattachés, forment un hétérocycle azoté contenant 3 à 5 atomes de carbone ou 2 à 4 atomes de carbone et un atome de soufre, et dans laquelle lesdits groupes alkyle, alcényle et alcynyle peuvent être substitués par des groupes hydroxyle alcoxyle inférieur, thio, alkyle inférieur-mercapto, amine, alkyle inférieur-amine, bis-(alkyle inférieur)-amine, des halogènes et des groupes nitro, et de leur sels, spécialement de leurs sels pharmaceutiquement acceptables d'acides et de bases, qui consiste à faire réagir, dans des conditions de formation d'amide, un composé aminé de formule II

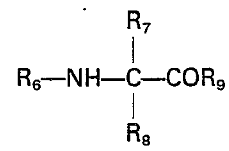

(II)

sur un dérivé acylant d'un acide de formule III

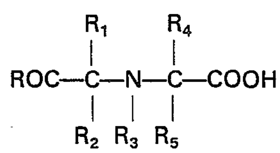

(III)

ou à réduire l'imine correspondante; ou par réaction, pour éliminer de l'halogénure d'hydrogène, d'un composé de formule VI

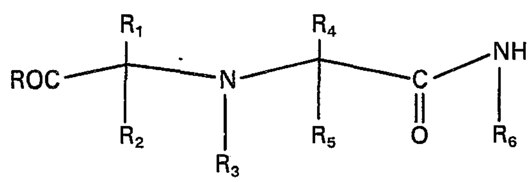

(VI)

sur un composé halogéné de formule VII

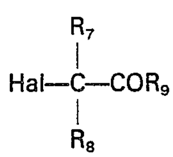

(VII)

et facultativement, par des réactions de substitution ou de conversion, à introduire divers substituants dans les produits; et facultativement, à en former des sels, spécialement des sels pharmaceutiquement acceptables d'acides et de bases.

2. Procédé selon la revendication 1, dans lequel R et $R_9$ sont indépendamment des groupes hydroxyle et alcoxyle inférieur,

$R_1$ et $R_2$ sont indépendamment des atomes d'hydrogène, des groupes alkyle inférieur, aryl-alkyle inférieur ayant de 7 à 12 atomes de carbone ou hétérocyclique-alkyle inférieur ayant de 6 à 12 atomes de carbone,

$R_3$, $R_4$, $R_5$, $R_7$ et $R_8$ sont des atomes d'hydrogène ou des groupes alkyle inférieur et

$R_6$ est un groupe hétérocyclique ou hétérocyclique-alkyle, et leur sels d'acides et bases pharmaceutiquement acceptables.

3. Procédé selon l'une des revendications 1 et 2, dans lequel $R_6$ est un groupe pyrrole, pyrroline, pyrrolidine, pyridine, dihydropyridine, pipéridine, morpholine, thiamorpholine, imidazole, imidazoline, imidazolidine, furanne, furfuryle, thiophène, benzimidazole, thiazole, thiazoline, thiazolidine, indole, quinoléine, isoquinoléine, tétrahydroquinoléine ou tétrahydroisoquinoléine.

4. Procédé selon l'une quelconque des revendications 1 à 3, dans lequel les groupes hétérocycliques ont un ou plusieurs substituants choisis dans le groupe formé des groupes alkyle inférieur, alcényle

inférieur, alcynyle inférieur, hydroxyle, alcoxyle inférieur, amine, alkyle inférieur-amine, bis-(alkyle inférieur)-amine, thiol, alkyle inférieur-mercapto, hydroxyalkyle inférieur, aminoalkyle inférieur, thioalkyle inférieur, nitro, des halogènes, des groupes trifluorométhyle, méthylènedioxy, uréido ou guanidino.

5. Procédé selon l'une quelconque des revendications 1 à 4, dans lequel $R_1$ est un groupe alkyle inférieur ou phényl-alkyle inférieur, $R_4$ est un groupe alkyle inférieur et $R_2$, $R_3$, $R_5$, $R_7$ et $R_8$ sont des atomes d'hydrogène.

6. Procédé selon l'une quelconque des revendications 1 à 4, dans lequel R et $R_9$ sont des groupes hydroxyle.

7. Procédé selon l'une quelconque des revendications 1 à 4, dans lequel R est un groupe éthoxyle et $R_9$ est un groupe hydroxyle.

8. Procédé selon l'une quelconque des revendications 1 à 7, dans lequel $R_1$ et $R_4$ sont chacun un groupe méthyle.

9. Procédé selon l'une quelconque des revendications 1 à 7, dans lequel $R_1$ est un groupe benzyle ou phénéthyl et $R_4$ est un groupe méthyle.

10. Procédé selon l'une quelconque des revendications 1 à 7, dans lequel $R_6$ est un groupe indolyléthyle.

11. Procédé selon l'une quelconque des revendications 1 à 7, dans lequel $R_1$ est un groupe benzyle.

12. Procédé selon l'une quelconque des revendications 1 à 7, dans lequel $R_1$ est un groupe phénéthyle.

13. Procédé selon l'une quelconque des revendications 1 à 4, dans lequel R est un groupe éthoxyle, $R_1$ est un groupe phénéthyle, $R_4$ est un groupe méthyle, $R_6$ est un groupe 2-(3-indolylméthyle) et $R_9$ est un groupe hydroxyle.

14. Procédé selon la revendication 13, dans lequel $R_2$, $R_3$, $R_5$, $R_7$ et $R_8$ sont chacun un atome d'hydrogène.

15. Procédé selon les revendications 1 et 2, dans lequel R est un groupe éthoxyle; $R_1$ est un groupe phénéthyle, $R_4$ est un groupe méthyle, $R_6$ est un groupe 3-pyridylméthyle, morpholinyle, indolyle, pyrrolidinyle ou indole-alkyle et $R_9$ est OH.

16. Procédé selon la revendication 15, dans lequel $R_2$, $R_3$, $R_5$, $R_7$ et $R_8$ sont chacun un atome d'hydrogène.

17. Procédé pour la préparation d'une composition pharmaceutique, consistant à formuler au moins un composé de la formule selon la revendication 1 en même temps que des véhicules pharmaceutiques usuels.